# FASCICULE DE BREVET EUROPEEN

(11) **EP 3 008 059 B1**
(45) Date de publication et mention de la délivrance du brevet: **19.12.2018**
(21) Numéro de dépôt: 14733376.9
(22) Date de dépôt: 13.06.2014
(51) Int. Cl.: C07D 407/04, C07D 493/04

(54) **PROCÉDÉ POUR LA PRÉPARATION D'ACÉTALS CYCLIQUES ALKYL À LONGUES CHAÎNES, À BASE DE SUCRES**
VERFAHREN ZUR HERSTELLUNG LANGKETTIGER VON CYCLISCHER ALKYLACETALE AUS ZUCKERN
METHOD FOR PREPARING LONG-CHAIN ALKYL CYCLIC ACETALS MADE FROM SUGARS

(30) Priorité: 14.06.2013 FR 1301375
(43) Date de publication de la demande: 20.04.2016
(73) Titulaire: Tereos Starch & Sweeteners Belgium, 9300 Aalst (BE); Université Claude Bernard Lyon 1, 69622 Villeurbanne Cedex (FR); Centre National de la Recherche Scientifique (C.N.R.S.), 75016 Paris (FR)
(72) Inventeur: GOZLAN, Charlotte, 93190 Livry-Gargan (FR); DUGUET, Nicolas, 69330 Meyzieu (FR); LEMAIRE, Marc, 69622 Villeurbanne Cedex (FR); QUENEAU, Yves, 69622 Villeurbanne Cedex (FR); REDL, Andreas, 9300 Aalst (BE)
(74) Mandataire: Chielens, Kristof
(86) Numéro de dépôt international: PCT/IB2014/062194
(87) Numéro de publication internationale: WO 2014/199345

(56) Documents cités:
- EP-A1- 0 019 999
- US-A- 3 484 459
- Neil Baggett ET AL: "SYNTHESIS OF SOME MONOACETALS OF 1,4-ANHYDRO-D-MANNITOL", Carbohydrate Research, 1 janvier 1983 (1983-01-01), pages 49-60, XP055078058, Extrait de l'Internet: URL:http://ac.els-cdn.com/S000862150090952 2/1-s2.0-S0008621500909522-main.pdf?_tid=1 d7e2f9e-16fa-11e3-94b0-00000aacb362&acdnat =1378475104_dbaae70aa8379c1e813b7e826f3902 1a [extrait le 2013-09-06]
- ALBERT L. RAYMOND ET AL: JOURNAL OF THE AMERICAN CHEMICAL SOCIETY, vol. 70, no. 8, 19 août 1948 (1948-08-19), pages 2785-2791, XP055078063, ISSN: 0002-7863, DOI: 10.1021/ja01188a045
- M. Csiba ET AL: "Liquid Crystalline 5,6-O-Acetals of L-Galactono-1,4-Lactone Prepared by a Microwave Irradiation on Montmorillonite", Tetahedron Letters, 1 janvier 1993 (1993-01-01), pages 1787-1790, XP055078175, Extrait de l'Internet: URL:http://ac.els-cdn.com/S004040390060779 7/1-s2.0-S0040403900607797-main.pdf?_tid=f bebc4d8-1931-11e3-8968-00000aab0f02&acdnat =1378719002_49e6d4ff77eb031c251bcbfbd10396 f3 [extrait le 2013-09-09]
- FANTON E ET AL: "Long-chain acetals derived from sucrose as a new class of surfactants", CARBOHYDRATE RESEARCH, PERGAMON, GB, vol. 298, no. 1-2, 20 février 1997 (1997-02-20), pages 85-92, XP004109759, ISSN: 0008-6215, DOI: 10.1016/S0008-6215(96)00300-X
- DUCLOS A ET AL: "A SIMPLE CONVERSION OF POLYOLS INTO ANHYDROALDITOLS", SYNTHESIS, GEORG THIEME VERLAG, STUTTGART, DE, vol. 10, 1 octobre 1994 (1994-10-01), pages 1087-1090, XP001056553, ISSN: 0039-7881, DOI: 10.1055/S-1994-25643

## Description

La présente invention concerne un procédé pour la préparation d'acétals cycliques alkyle à chaînes longues, à base de sucres.

Dans la littérature scientifique et technique, les molécules tensioactives à base de sucre sont bien connues. Parmi elles, les esters d'acides gras de saccharose, les esters de sorbitan et les alkyles polyglucosides à longues chaînes ont été largement utilisés dans l'alimentation, les soins personnels, applications cosmétiques et pharmaceutiques. Également dans des applications de nettoyants domestiques et industrielles, et comme lubrifiants certains de ces tensioactifs ont trouvé une large acceptation.
Malgré leur utilisation et leur acceptation répandue, il est bien connu que les tensio-actifs à base d'esters ne sont stables que sur une étendue limitée de pH, tandis que les glucosides d'alkyle sont stables dans des conditions alcalines et neutres, mais pas dans des conditions acides.

D'autres inconvénients sont liés aux procédés utilisés pour l'obtention de ces dérivés. Dans le cas des glucosides d'alkyle supérieur, une trans-acétalisation est nécessaire. L'utilisation d'installations assez compliquées et coûteuses est nécessaire afin d'obtenir un produit suffisamment pur. Dans le cas des esters à base de sucres, notamment les esters de sorbitane, on a besoin de solvants coûteux et toxiques, ou des températures de réaction élevées pour obtenir les produits avec un rendement suffisamment haut.

Afin d'améliorer la stabilité acide des composés tensioactifs à base de sucre, un éther d'alcool de sucre a été proposé récemment dans le document WO 2012/148530. Cette demande décrit un procédé pour préparer ces éthers de polyols par lequel une masse en fusion de polyol est mise à réagir avec un aldéhyde alkyle supérieur dans des conditions d'alkylation réductrice. Ici aussi, des conditions difficiles et extrêmes sont nécessaires, ceci en combinaison avec un équipement à haute pression afin de réaliser la réaction d'alkylation réductrice. Afin d'obtenir les produits désirés un excès d'alcool de sucre par rapport à l'aldéhyde est jugé nécessaire. Ceci résulte en une consommation d'énergie par mole d'éther d'alcool de sucre qui est élevée.

Un autre groupe de molécules tensioactives à base de sucre est représenté par les d'acétals cycliques alkyle à chaînes longues de sucres, comme décrit dans plusieurs publications scientifiques et techniques.
Dans Carbohydrate Research (1997) p.85-92, des acétals cycliques alkyles supérieurs de saccharose sont décrits, ainsi que des méthodes pour les obtenir. Les acétals ainsi obtenus pourraient présenter un intérêt dans le domaine des détergents car ces produits sont stables en milieu basique et neutre, ce qui est en contraste avec les dérivés d'esters. En outre, ils ont montré des valeurs CMC intéressantes. En OPPI Briefs (1998) p.460-464, une méthode améliorée a été divulguée pour la préparation de tels composés à base de saccharose.

Dans le document US 6251937 (FR2761991) et JAOCS (1994) p.705-710, les acétals cycliques alkyles supérieurs des dérivés de l'acide gluconique sont décrits qui présentent des propriétés tensioactives dans des milieux basiques et neutres. En même temps, ils ont également montré une forte capacité d'hydrolyse dans un milieu acide.

Dans le brevet EP 0 019 999, la préparation des acétals cycliques alkyle supérieurs de dérivés de sucre, en particulier de sorbitol, est divulguée. Ainsi un procédé amélioré est proposé utilisant l'acide acétique en tant que milieu réactionnel. Cette réaction produit un acétal d'alkyle de sorbitol partiellement substitué de groupes d'acétate. Dans ce même document, il est fait référence au brevet US 4,031,112. Dans ce dernier document, il est mentionné que les conditions de réaction qui y sont décrites peuvent également être utilisées pour préparer des acétals alkyles à chaînes longues de sorbitol. Cependant, il a été constaté, comme mentionné dans le brevet EP 0 019 999, que les conditions décrites aboutissent à la vaste décomposition des produits et réactifs, par lequel le rendement et la qualité du produit sont devenu commercialement inacceptable.

Dans le brevet US 3,484,459, on fait référence à la préparation d'acétals cycliques de sorbitane. Dans ce document, on mentionne une large gamme d'aldéhydes et de cétones comme réactifs potentiels. Ces réactions d'acétalisation sont utilisées pour récupérer le 1,4- sorbitane résiduel à partir d'un mélange d' hexitanes, après séparation des acétals purs par distillation fractionnée. Le sorbitane acétal ainsi obtenu est hydrolysé, et le 1,4-sorbitane est récupéré par cristallisation. L'acétalisation est ainsi réalisée avec un large excès de réactif, en utilisant des temps de réaction longs. Les conditions utilisées ne sont pas très attrayantes d'un point de vue du processus.

De ce qui précède, il est clair que les produits et /ou procédés décrits à l'égard des acétals cycliques d'alkyles supérieurs à base de sucre montrent un certain nombre de lacunes. En dehors de l'éther de polyol décrit dans WO 2012/148530, toutes les autres molécules tensio-actives à base de sucre ne sont pas ou insuffisamment stables dans des conditions acides, tandis que dans la plupart des cas, les procédés utilisent des solvants ou des conditions de réaction qui ne sont pas sûres d'un point de vue environnemental, et /ou qui consomment beaucoup d'énergie et /ou ne sont pas rentable d'un point de vue industriel.

Par conséquent, il est évident qu'il demeure un besoin non satisfait de disposer d'acétals cycliques alkyles à longues chaînes à base de sucre qui montrent une stabilité améliorée à des conditions acides, en combinaison avec de bonnes propriétés d'émulsifiantes. En outre, il demeure également un besoin de disposer de procédés pour préparer ces composés, procédés qui sont acceptables pour l'environnement, qui sont avantageux dans la consommation d'énergie et qui sont faciles à mettre en oeuvre industriellement.

Le but de l'invention est obtenu en fournissant un procédé pour la préparation d'un acétal cyclique alkyle à chaîne longue à base de sucres, dans lequel le procédé comprend les étapes suivantes:
- déshydratation d'un hexitol en formant un monoanhydro hexitol;
- la réaction du substrat monoanhydro hexitol obtenu avec un réactif d'aldéhyde alkyle contenant de 5 à 18 atomes de carbone, par l'intermédiaire d'une réaction d'acétalysation dans un rapport de substrat/réactif compris entre 5:1 et 1:1, ou avec un acétal de di-alkyle d'un aldéhyde alkyle contenant de 5 à 18 atomes de carbone, par l'intermédiaire d'une réaction de trans-acétalysation dans un rapport de substrat/réactif compris entre 1:1 et 1:3, préférentiellement, en présence d'un catalyseur acide et/ou dans un environnement qui est exempt de solvant ou qui se compose d'un solvant polaire non-aqueux;
- la récupération de l'acétal alkyle à longue chaîne d' hexitane à partir du mélange obtenu.
Typiquement, par « alkyle à longue chaîne » on entend un radical alkyle comprenant préférentiellement, de 5 à 18 atomes de carbones, préférentiellement, 8 à 12 atomes de carbones.
Dans un procédé préféré selon l'invention, ledit hexitol est choisi dans le groupe constitué de: sorbitol, le mannitol, le galactitol et l'iditol. Le sorbitol est l'hexitol préféré.
Dans un procédé plus préféré de l'invention, ledit réactif d'aldéhyde alkyle contient de 8 à 12 atomes de carbone.
Lors de l'exécution de la réaction d'acétalysation avec un réactif aldéhyde, la réaction peut être effectuée avec ou sans solvant. Lors de l'utilisation d'un solvant, et selon un procédé avantageux selon l'invention, ledit solvant polaire non-aqueux est choisi dans le groupe constitué par: le DMF, le DMSO, le DMA, l'acétonitrile, le THF, le méthyl THF, ou de l'acétate d'éthyle.

Dans un procédé particulier conforme à l'invention ledit acétal alkyle à longue chaîne d' hexitane est récupéré par séparation.
Dans un procédé préféré selon l'invention, ledit substrat monoanhydro hexitol est du 1,4 sorbitane purifié.
Dans un procédé plus particulièrement selon l'invention, ledit acétal alkyle à longue chaîne d'hexitane est composé de quatre diastéréoisomères.

Cette invention va maintenant être décrite plus en détail et illustrée par des graphiques et des exemples qui doivent être considérés comme ne pas limitant la portée de l'invention en tant que telle et telle qu'elle est exprimée dans les revendications suivantes ci-dessous, dans lequel les numéros de référence sont utilisés pour désigner les dessins annexés dans lesquels :
- *figure 1**: représente un chromatogramme du mélange de réaction obtenu au cours de la réaction de déshydratation;*
- *figure 2**: représente un chromatogramme du mélange de réaction obtenu par trans-acétalisation sans solvant selon l'exemple 8.*
Selon cette invention, il a été constaté d'une façon surprenante que les acétals cycliques alkyle à longue chaîne à base de sucre peuvent être obtenus par un procédé qui comprend les étapes suivantes:
- la déshydratation d'un hexitol dans un monoanhydro hexitol,
- la réaction du substrat monoanhydro hexitol obtenu avec un réactif d'aldéhyde alkyle contenant de 5 à 18 atomes de carbone, par l'intermédiaire d'une réaction d'acétalysation dans un rapport de substrat/réactif entre 5:1 et 1:1, ou avec un acétal de di-alkyle d'un aldéhyde alkyle contenant de 5 à 18 atomes de carbone, par l'intermédiaire d'une réaction de trans-acétalysation dans un rapport de substrat/réactif entre 1:1 et 1:3, en présence d'un catalyseur acide et dans un environnement qui est exempt de solvant ou qui se compose d'un solvant polaire non-aqueux,
- et à récupérer l'acétal alkyle à longue chaîne d'hexitane à partir du mélange obtenu.

Hexitols typiques sont le sorbitol, le mannitol, le galactitol et l'iditol, par laquelle le sorbitol est de loin le plus abondant.

La formation de monoanhydro sorbitol a déjà été décrite dans plusieurs publications. Ainsi différentes méthodes ont été décrits pour l'obtention de ce composé intermédiaire.

Dans un mode de réalisation, le sorbitol est dissous dans l'eau en présence d'un catalyseur acide et chauffé dans des conditions atmosphériques pendant un temps suffisant pour obtenir la teneur maximale de 1,4-sorbitane. Un tel procédé est décrit dans Acta Chemical Scandinavica B (1981) p.441-449. Aussi des procédés ont été divulgués où la réaction est effectuée sous pression réduite (US 2,390,395 et US 2007173651) ou sous pression d'hydrogène modérée (US2007173654). Dans cette demande de brevet US2007173654 un co-catalyseur de métal noble est utilisé ce qui aboutissait à des concentrations assez élevées de l'isosorbide, à la place du 1,4-sorbitane. 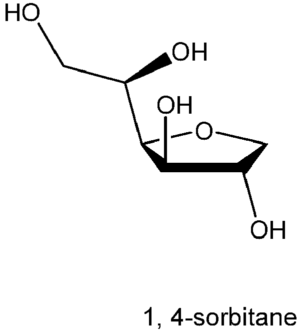 Selon la demande de brevet actuel, il a été constaté que selon une forme de réalisation préférée, le produit intermédiaire 1,4-sorbitane pourrait être obtenu avec un bon rendement par traitement d'une masse fondue de sorbitol avec un catalyseur acide solide dans une atmosphère d'hydrogène à une pression de 20 à 50 bar, ceci à une température de réaction qui peut varier entre 120 et 170 ° C, pendant une période de temps suffisante afin d'obtenir un rendement optimal de sorbitane. Les températures de réaction préférées sont comprises entre 130 et 140 ° C.

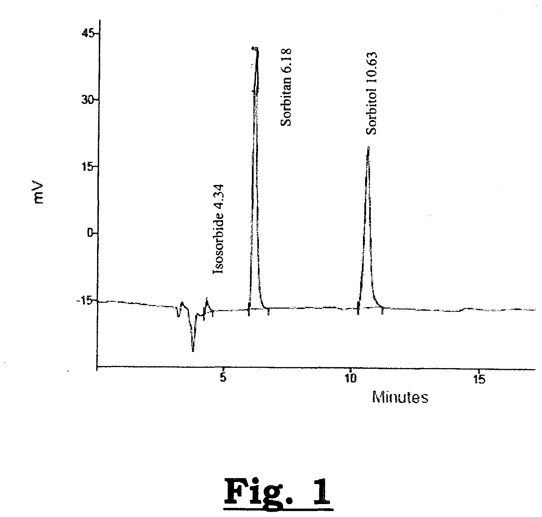

Le mélange réactionnel ainsi obtenu est constitué de 1,4-sorbitane, de sorbitol qui n'a pas réagi, de l'isosorbide et de quantités mineures de sous-produits, comme illustré sur le chromatogramme représenté sur la fig. 1. Un des avantages observés ainsi est la baisse du niveau de coloration, ceci en contraste avec les procédés antérieurs classiques.
Ce mélange réactionnel peut ensuite être utilisé dans l'étape suivante tel quel, mais il est préférable de récupérer et purifier le 1,4-sorbitane de ce mélange et à recycler le reste vers l'étape de déshydratation. Dans un mode de réalisation particulier, le 1,4-sorbitane est récupéré et purifié par cristallisation. Dans un autre mode de réalisation préféré le 1,4-sorbitane est récupéré et purifié par moyen d'un processus chromatographique. Ce 1,4-sorbitane purifié est utilisé de préférence en tant que substrat pour la réaction d'acétalysation.

La réaction d'acétalysation peut être réalisée avec un réactif d'aldéhyde alkyle, dans lequel le réactif aldéhyde contient de 5 à 18 atomes de carbone. Ces aldéhydes peuvent être choisis parmi les aldéhydes linéaires ou ramifiés, et parmi les aldéhydes aliphatiques ou aromatiques. Dans un mode de réalisation préféré, les aldéhydes alkyle contiennent de 8 à 12 atomes de carbone. Certains représentants typiques des aldéhydes sont: le pentanal, l'hexanal, l'heptanal, l'octanal, le nonanal, le decanal et le dodécanal.
L'acétalysation peut également être effectuée en utilisant les acétals de di-alkyle des aldéhydes correspondants, les acétals de di-méthyle et les acétals de di-éthyle étant préférés.

Lors de l'exécution de la réaction d'acétalysation avec un réactif d'aldéhyde, la réaction peut être effectuée avec ou sans solvant. Lors de l'utilisation d'un solvant, celui peut être choisi parmi les solvants polaires tels que le DMF, le DMSO, DMA, l'acétonitrile, le THF, THF méthyle ou l'acétate d'éthyle. Du travail expérimental étendu a permis de sélectionner ainsi des conditions assurant des taux de conversion et des rendements optimales. Les meilleurs résultats ont été obtenus lorsque le rapport molaire du substrat au réactif est entre 5:1 et 1:1, de préférence compris entre 4:1 et 1:1, et de manière plus préférée entre 3:1 et 2:1.

Lorsque la réaction est effectuée sans solvant, le 1,4-sorbitane est d'abord chauffé entre 90 et 110 ° C, puis le réactif aldéhyde est ajouté lentement, suivi de l'addition du catalyseur. Les catalyseurs acides utilisés peuvent être choisis parmi les acides solides ou liquides, organiques ou inorganiques, des acides solides étant préférés. En particulier, les acides préférés sont choisis parmi l'acide para-toluène sulfonique, l'acide méthane sulfonique et CSA (acide sulfonique de camphre).

De plus des réactions de trans-acétalysation peuvent être réalisées en présence ou en absence d'un solvant afin d'obtenir des acétals cycliques alkyle à chaînes longues, à base de sucres. Dans le cas où un solvant est utilisé, il est préférable d'utiliser de l'alcool correspondant au réactif acétal utilisé. A partir du travail expérimental il a été constaté que dans les réactions de trans-acétalisation, des rendements et des taux de conversion optimales ont été obtenus lorsque le rapport molaire du substrat au réactif est entre 1 :1 et 1 :3, et de préférence entre 2 :3 et 2 :5. Les mêmes catalyseurs sont utilisé que ceux utilisés dans les réactions d'acétalysation.

Lors de la réalisation des réactions d'acétalysation, les mélanges réactionnels sont chauffés à des températures variant entre 70 ° C et 100 ° C, en fonction des réactifs et des solvants utilisés. Le temps de réaction est déterminé par le degré de conversion atteint.

Les mélanges de réaction bruts ainsi obtenus sont ensuite traités afin de récupérer les acétals alkyle d'hexitane selon l'invention. La récupération est faite par des méthodes de séparation généralement connus dans l'état de la technique. Les méthodes typiques qui peuvent être utilisés sont, entre autre, l'extraction, la séparation chromatographique et la cristallisation.

Les compositions d'acétal de sorbitane obtenus par les procédés décrits ci-dessus sont composé de 4 diastéréoisomères. Deux diastéréoisomères correspondent avec une sorbitane 5,6-acétal et les deux autres correspondent à une sorbitane 3,5-acétal. Ainsi R est une chaîne aliphatique linéaire en C4 à C17.

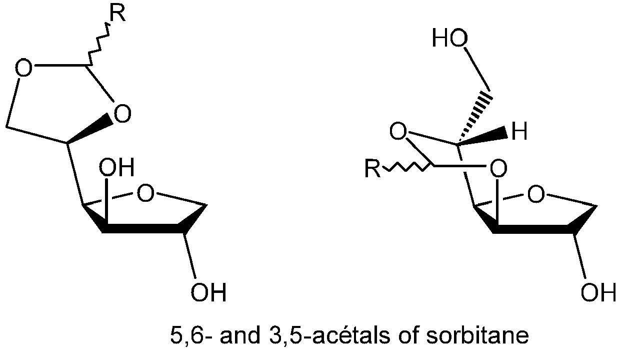

Des tests de stabilité des compositions d'acétals d'alkyle sorbitane selon l'invention ont été réalisé dans de l'éthanol et de l'eau à différentes valeurs de pH. Ainsi il a été constaté que moins de 1% a été hydrolysé dans l'éthanol au bout de 3 heures à un pH = 1 et à 80 ° C.

Des tests dans l'eau ont été effectué à 3 valeurs de pH: 7, 5 et 1. A pH = 5 et pH=7 pas de dégradation substantielle a été observée après 48 heures, à des températures de 20 ° C et 40 ° C. A un pH = 1, au bout de 4 heures à 20 ° C, 40% est hydrolysé, tel que déterminé par HPLC. En comparaison avec les acétals, décrit dans JAOCS 1994, p.705-710, il s'agit d'une amélioration nette de la stabilité acide par rapport à ces composés.

Les compositions ainsi obtenues peuvent donc être utilisés comme agent tensioactif non ionique, comme émulsifiant, comme lubrifiant ou agent de dispersion dans une large gamme d'applications alimentaires et non alimentaires.

Sans limiter la portée de l'invention, l'invention va maintenant être davantage illustrée à l'aide d'un certain nombre d'exemples décrivant les méthodes de préparation de ces dérivés.

### Exemple 1 :

### La déshydratation du sorbitol:

D-sorbitol (20 g, 110 mmol) et de 0,1% en moles d'acide camphosulfonique sont ajoutés dans un autoclave de 150 ml en acier inoxydable. Le réacteur est hermétiquement fermé, purgé trois fois avec de l'hydrogène et ensuite l'hydrogène a été introduit jusqu'à une pression de 50 bars. Le système est ensuite chauffé à 140 °C et agité avec un agitateur mécanique pendant 15 heures. Après refroidissement à température ambiante, la pression d'hydrogène a été libérée et la mousse blanche a été diluée dans de l'éthanol (200 ml) pour obtenir un mélange homogène jaune. Le solvant est évaporé sous pression réduite et le résidu est ensuite cristallisé à partir de méthanol froid et filtré sous vide. Le matériau cristallin a été lavé avec du méthanol froid pour donner le 1,4-sorbitane (5,88 g, 35% sur théorique) comme un solide blanc. La pureté est de > 98%, tel que déterminé par HPLC, tandis que les cristaux ont montré un point de fusion de 113-114 ° C. Le degré de conversion de la réaction a été déterminée à 73%, grâce à quoi on obtient un mélange de sorbitol, de 1,4-sorbitanee, d'isosorbide et de quelques sous-produits en quantité très limité, de sorte que le rapport du 1,4-sorbitane: isosorbide a été déterminé comme étant de 80: 20.

### Exemple 2:

### Acétalysation de sorbitanee dans le DMF:

Dans un tube scellé, 1,4 sorbitane (X) (0,5 g, 3 mmol) a été dissous dans du DMF (1,4 ml). Valeraldehyde (Y) (107µl, 1 mmol) a été ajouté goutte par goutte sous argon suivi par l'addition d'acide camphosulfonique (10 mg, 10% p / p) avant de fermer le tube. Le mélange est chauffé à 95 °C et sous agitation magnétique. Après 15 heures, le mélange réactionnel foncé a été refroidi et le solvant évaporé sous pression réduite. Un degré de conversion de 95% a été atteint. Le résidu a été dilué dans de l'acétate d'éthyle et l'excédent de 1,4 sorbitane a été filtré et lavé avec de l'acétate d'éthyle. Le filtrat a été concentré sous pression réduite. Le résidu est purifié par chromatographie flash (EtOAc: cyclohexane 80:20 à 100:0) pour donner le sorbitane acétal (0,22 g, 89% de rendement isolé) comme une huile incolore. Le HPLC a révélé un mélange de 4 diastéréoisomères.

### Exemple 3:

Dans cet exemple, différents ratios de sorbitane contre le réactif aldéhyde ont été testés. Les mêmes conditions de réaction que dans l'exemple 2 ont été utilisés, mais le rapport sorbitane: aldéhyde a été varié entre 1 :1 et 3 :1. Les résultats sont présentés dans le tableau 1, ci-dessous.

**Tableau 1: Effet du rapport sorbitane: aldéhyde sur le degré de conversion et le rendement isolé**

| **Rapport X/Y** | **Conversion** | **Rendement isolé (% en poids** |
|---|---|---|
| 1 :1 | 96% | 62% |
| 2 :1 | 81% | 83% |
| 3 :1 | 95% | 89% |

### Exemple 4:

Avec un ratio sorbitane: aldéhyde de 3:1 de différents réactifs aldéhydes ont été utilisés pour fournir des produits de réaction acétal sorbitane. Les mêmes conditions de réaction et les mêmes étapes de purification comme dans l'exemple 2 ont été utilisés.

Les résultats sont présentés dans le tableau 2.

**Tableau 2:**

| **Aldéhyde** | **Conversion** | **Rendement isolé** |
|---|---|---|
| Hexanal | 100% | 98% |
| Octanal | 89% | 95% |
| Decanal | 69% | 85% |
| Dodecanal | 61% | 80% |

### Exemple 5:

Outre l'utilisation du DMF comme solvant, aussi d'autres solvants ont été utilisés pour préparer les compositions de sorbitane acétal. Ici aussi, les mêmes réactifs ont été utilisés et la même procédure a été suivie comme dans l'exemple 2, sauf que les températures de réaction étaient aux environs de 80 ° C. Les résultats sont présentés dans le tableau 3.

**Tableau 3:**

| **Solvant** | **Conversion** | **Rendement isolé** |
|---|---|---|
| Acétonitrile | 100% | n.d. |
| Acétate d'éthyle | 98% | n.d. |
| DMF | 83% | 83% |

### Exemple 6:

### Acétalysation de sorbitane sans solvant:

Dans un tube scellé, 1,4 sorbitane (X) (0,5 g, 3 mmol) a été chauffé à 95 ° C. Valeraldehyde (Y) (107µl, 1 mmol) a été ajouté goutte par goutte, sous argon, puis de l'acide camphosulfonique (10 mg, 10% p / p) avant de refermer le tube. Le mélange est chauffé à 95 °C sous agitation magnétique. Après 15 heures, le mélange réactionnel foncé a été refroidi et dilué dans l'acétate d'éthyle (2 ml) et le solvant est ensuite évaporé sous pression réduite. Un degré de conversion de 80% a été obtenu. Le résidu a été à nouveau dilué dans l'acétate d'éthyle et l'excédent de 1,4 sorbitane a été filtré et lavé avec de l'acétate d'éthyle. Le filtrat a été concentré sous pression réduite. Le résidu est purifié par chromatographie flash (EtOAc: cyclohexane 80:20 à 100:0) pour donner l'acétal de sorbitane (0,13 g, 54% de rendement isolé) comme une huile incolore. Le HPLC a révélé un mélange de 4 diastéréoisomères.

### Exemple 7:

### Trans-acétalysation de sorbitane dans l'éthanol:

Dans un ballon à fond rond du 1,4-sorbitane (0,5 g, 3 mmol) a été dissous dans de l'éthanol (7,5 ml) et le 1,1-diethoxypentane (1,15 ml, 6 mmol) a été ajouté sous un flux d'argon , puis de l'acide camphosulfonique (50 mg; 10% p/p). Le mélange est chauffé à 80 ° C et sous agitation magnétique. Après 3 heures, le mélange a été neutralisé et concentré sous pression réduite. Le résidu a été purifié par chromatographie flash (acétate d'éthyle / cyclohexane 80:20 à 100:0) pour donner l'acétal de sorbitane (0,43 g, 66% de rendement isolé) comme une huile incolore. Le HPLC a révélé un mélange de 4 diastéréoisomères.

### Exemple 8:

### Trans-acétalysation de sorbitane sans solvant:

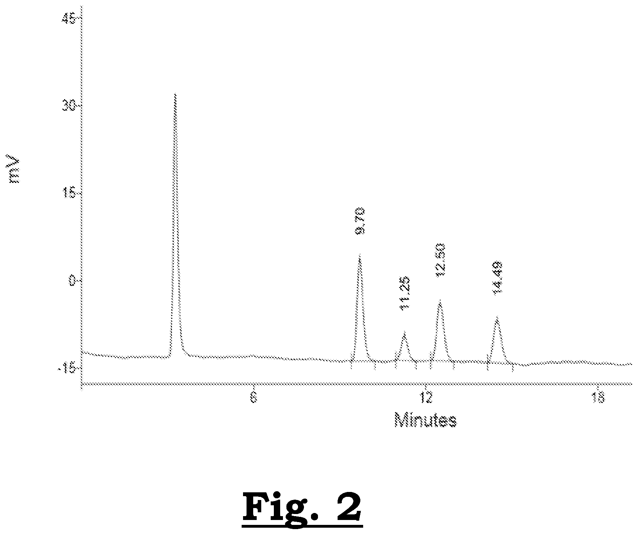

Dans un ballon à fond rond du 1,4-sorbitane (0,5 g, 3 mmol) et du 1,1-diethoxypentane (1,1-DEP) (1,15 ml, 6 mmol) (rapport molaire 1:2) ont été ajoutés sous flux d'argon, puis de l'acide camphosulfonique (50 mg; 10w / w%). Le mélange est chauffé à 80 ° C et sous agitation magnétique. Après 3 heures, le mélange a été directement purifié par chromatographie flash (acétate d'éthyle / cyclohexane 80:20 à 100:0) pour donner l'acétal de sorbitane (0,517 g, 73% de rendement isolé) comme une huile incolore. Le HPLC a révélé un mélange de 4 diastéréoisomères. (Fig. 2)

### Exemple 9:

Trans-acétalysations sans solvant ont été réalisées en utilisant différents rapports molaires, de différents réactifs (1,1-diméthoxypentane), de différentes températures de réaction et de différents temps de réaction, le catalyseur étant la même. La purification des mélanges de réaction a été réalisée par chromatographie flash, comme dans l'exemple 8.

Les résultats sont donnés dans le tableau 4.

**Tableau 4:**

| **Réactif** | **Sorbitane/réactif ratio** | **Temps (h)** | **Température** | **Conversion** | **Rendement isolé** |
|---|---|---|---|---|---|
| 1,1-DMP | 1:1 | 15 | 70°C | 99% | 66% |
| 1,1-DEP | 1:1 | 15 | 70°C | 81% | 66% |
| 1,1-DEP | 1:1 | 15 | 80°C | - | 49% |
| 1,1-DEP | 1:2 | 3 | 80°C | 80% | 73% |

## Revendications

1. Procédé pour la préparation d'acétals cycliques alkyle à chaînes longues, à base de sucres, **caractérisé en ce que** le procédé comprend les étapes suivantes consistant à:
- la déshydratation d'un hexitol dans un substrat monoanhydro hexitol;
- la réaction du substrat monoanhydro hexitol obtenu avec un réactif d'aldéhyde alkyle contenant de 5 à 18 atomes de carbone, par l'intermédiaire d'une réaction d'acétalysation dans un rapport molaire de substrat/réactif compris entre 5:1 et 1:1, ou avec un acétal de di-alkyle d'un aldéhyde alkyle contenant de 5 à 18 atomes de carbone, de préférence ledit di-alkyle étant un di-méthyle ou di-éthyle, par l'intermédiaire d'une réaction de trans-acétalysation dans un rapport molaire de substrat/réactif compris entre 1:1 et 1:3, en présence d'un catalyseur acide et dans un environnement qui est exempt de solvant ou qui se compose d'un solvant polaire non-aqueux;
- la récupération de l'acétal alkyle à longue chaîne d'hexitane à partir du mélange obtenu.

2. Procédé selon la revendication 1, dans lequel l'hexitol est choisi dans le groupe comprenant: le sorbitol, le mannitol, le galactitol ou l'iditol.

3. Procédé selon la revendication 2, dans lequel l'hexitol est le sorbitol.

4. Procédé selon l'une quelconque des revendications précédentes, dans lequel ledit réactif d'aldéhyde alkyle contient de 8 à 12 atomes de carbone.

5. Procédé selon l'une quelconque des revendications précédentes, dans lequel ledit solvant polaire non-aqueux est choisi dans le groupe constitué par: le DMF, le DMSO, le DMA, l'acétonitrile, le THF, le méthyl THF, ou de l'acétate d'éthyle.

6. Procédé selon l'une quelconque des revendications précédentes, dans lequel l'acétal alkyle à longue chaîne d'hexitane est récupéré par séparation.

7. Procédé selon l'une quelconque des revendications 3 à 6, dans lequel ledit substrat monoanhydro hexitol est du 1,4 sorbitane purifié.

8. Procédé selon la revendication 7, dans lequel ledit acétal alkyle à longue chaîne d'hexitane est composé de quatre diastéréoisomères.

## Patentansprüche

1. Verfahren zur Herstellung von langkettigen cyclischen Alkylacetalen auf Basis von Zuckern, **dadurch gekennzeichnet, dass** das Verfahren die folgenden Schritte umfasst:
- Dehydratisierung eines Hexitols zu einem Monoanhydrohexitol-Substrat;
- Umsetzung des erhaltenen Monoanhydrohexitol-Substrats mit einem Alkylaldehyd-Reagens mit 5 bis 18 Kohlenstoffatomen durch eine Acetalisierungsreaktion in einem Substrat/Reagens-Molverhältnis zwischen 5:1 und 1:1 oder mit einem Dialkylacetal eines Alkylaldehyds mit 5 bis 18 Kohlenstoffatomen, wobei es sich bei dem Dialkyl vorzugsweise um ein Dimethyl oder Diethyl handelt, durch eine Umacetalisierungsreaktion in einem Substrat/Reagens-Molverhältnis zwischen 1:1 und 1:3 in Gegenwart eines Säurekatalysators und in einer Umgebung, die lösungsmittelfrei ist oder aus einem nichtwässrigen polaren Lösungsmittel besteht;
- Gewinnen des langkettigen Hexitanalkylacetals aus dem erhaltenen Gemisch.

2. Verfahren nach Anspruch 1, bei dem das Hexitol aus der Gruppe umfassend Sorbitol, Mannitol, Galactitol und Iditol ausgewählt wird.

3. Verfahren nach Anspruch 2, bei dem es sich bei dem Hexitol um Sorbitol handelt.

4. Verfahren nach einem der vorhergehenden Ansprüche, bei dem das Alkylaldehyd-Reagens 8 bis 12 Kohlenstoffatome enthält.

5. Verfahren nach einem der vorhergehenden Ansprüche, bei dem das nichtwässrige polare Lösungsmittel aus der Gruppe bestehend aus DMF, DMSO, DMA, Acetonitril, THF, Methyl-THF oder Essigsäureethylester ausgewählt wird.

6. Verfahren nach einem der vorhergehenden Ansprüche, bei dem das langkettige Hexitanalkylacetal durch Abtrennung gewonnen wird.

7. Verfahren nach einem der Ansprüche 3 bis 6, bei dem es sich bei dem Monoanhydrohexitol-Substrat um gereinigtes 1,4-Sorbitan handelt.

8. Verfahren nach Anspruch 7, bei dem das langkettige Hexitanalkylacetal aus vier Diastereoisomeren besteht.

## Claims

1. Method for preparing long-chain alkyl cyclic acetals made from sugars, **characterized in that** the method comprises the following steps consisting of:
- dehydrating a hexitol in a monoanhydrohexitol substrate;
- reacting the monoanhydrohexitol substrate obtained with an alkyl aldehyde reagent containing 5 to 18 carbon atoms, by means of an acetalization reaction with a substrate/reagent molar ratio of between 5:1 and 1:1, or with a dialkyl acetal of an alkyl aldehyde containing 5 to 18 carbon atoms, said dialkyl preferably being a dimethyl or diethyl, by means of a transacetalization reaction with a substrate/reagent molar ratio of between 1:1 and 1:3, in the presence of an acid catalyst and in an environment that is free of solvent or that consists of a non-aqueous polar solvent;
- collecting the long-chain alkyl acetal of hexitan from the mixture obtained.

2. Method according to Claim 1, wherein the hexitol is selected from the group comprising: sorbitol, mannitol, galactitol and iditol.

3. Method according to Claim 2, wherein the hexitol is sorbitol.

4. Method according to any one of the preceding claims, wherein said alkyl aldehyde reagent contains 8 to 12 carbon atoms.

5. Method according to any one of the preceding claims, wherein said non-aqueous polar solvent is selected from the group consisting of: DMF, DMSO, DMA, acetonitrile, THF, methyl THF and ethyl acetate.

6. Method according to any one of the preceding claims, wherein the long-chain alkyl acetal of hexitan is collected by separation.

7. Method according to any one of Claims 3 to 6, wherein said monoanhydrohexitol substrate is purified 1,4-sorbitan.

8. Method according to Claim 7, wherein said long-chain alkyl acetal of hexitan is composed of four diastereoisomers.
